# EUROPÄISCHE PATENTSCHRIFT

(11) **EP 1 634 602 B1**
(45) Veröffentlichungstag und Bekanntmachung des Hinweises auf die Patenterteilung: **22.09.2010**
(21) Anmeldenummer: 05014057.3
(22) Anmeldetag: 29.06.2005
(51) Int. Cl.: A61K 36/53, A61P 25/00, A61P 25/24, A61P 15/12, A61P 1/00

(54) **Verwendung von Pflanzen der Gattung Sideritis zur Vorbeugung und Beeinflussung von Störungen, die mit einer veränderten serotoninergen Neurotransmission verbunden sind**
Use of plants of the genus Sideritis for preventing and influencing disturbances correlated with a changed serotoninergic neurotransmission
Utilisation de plantes du genre Sideritis pour prévenir et influencer des désordres associés avec un neurotransmission serotonique changé.

(30) Priorität: 02.07.2004 DE 102004032165
(43) Veröffentlichungstag der Anmeldung: 15.03.2006
(73) Patentinhaber: IBAM GbR, 79211 Denzlingen (DE)
(72) Erfinder: Knörle, Rainer, 79263 Simonswald (DE); Schnierle, Peter, 79114 Freiburg (DE)
(74) Vertreter: Stoppkotte, Cornelia

(56) Entgegenhaltungen:
- E.A. ABOUTABL ET AL.: "Phytochemical and pharmacological studies on Sideritis taurica Stephan ex Wild" JOURNAL OF ETHNOPHARMACOLOGY, Bd. 82, 2002, Seiten 177-184, XP009060299 Ireland
- MARGARITA HERNANDEZ-PEREZ ET AL.: "Evaluation of the antiinflammatory and analgesic activity of Siderits canariensis var. pannosa in mice" JOURNAL OF ETHNOPHARMACOLOGY, Bd. 81, 2002, Seiten 43-47, XP009060298 Ireland
- EVA KASSI ET AL.: "Greek Plant Extracts exhibit selective estrogen receptor modulator (SERM)-like properties" J. AGRIC. FOOD CHEM., Bd. 52, 2004, Seiten 6956-6961, XP009060410 American Chemical Society
- YUSUF ÖZTÜRK ET AL.: "Effects of Extracts from certain Sideritis Species on Swimming performance in Mice." PHYTOTHERAPY RESEARCH, Bd. 10, 1996, Seiten 70-73, XP009060306 United Kingdom
- ESPLUGUES J. ET AL.: "Activité pharmacodynamique de Sideritis mugronensis" PLANTES MÉDICINALES ET PHYTOTHÉRAPIE, Bd. 2, 1982, Seiten 137-146, XP009060468

## Beschreibung

Die vorliegende Erfindung betrifft die Verwendung von Extrakten von Pflanzen der Gattung Sideritis und/oder deren Pflanzenteilen zur Vorbeugung und/oder Beinflussung von Störungen, die mit einer veränderten serotoninergen Neurotransmission verbunden sind, wobei es sich bei den Störungen um depressive Störungen, Panikattacken, generalisierte Angststörung, Zwangssyndrom, klimakterische Beschwerden und Eßstörungen wie Bulimie handelt.

Serotonin (5-Hydroxytryptamin, 5-HT) ist ein biogenes Amin, das beim Menschen im Zentralnervensystem (Bulbus olfactorius, Diencephalon, insbesondere Hypophyse und Mesencephalon) und vor allem in den enterochromaffinen Zellen des Magen-Darm-Trakts (zu 90%) synthetisiert wird. Im Blut wird es in den Thrombocyten transportiert.

Es handelt sich um ein Gewebshormon das im Gehirn als Neurotransmitter wirksam ist.

Ausgangspunkt der Serotonin-Biosynthese ist die für den Menschen essentielle Aminosäure L-Tryptophan, die von den Serotonin-produzierenden Zellen aus dem Blut aufgenommen wird.

Ins Gehirn gelangt Tryptophan über ein Transportsystem, das es mit den verzweigtkettigen Aminosäuren (Valin, Leucin und Isoleucin), Phenylalanin und Tyrosin teilt.

Durch Hydroxylierung am Indolring durch eine mischfunktionelle Oxygenase wird zuerst 5-Hydroxytryptophan gebildet. Dann wird 5-Hydroxytryptophan in einer Pyridoxalphosphat-abhängigen Reaktion zu 5-Hydroxytryptamin decarboxyliert.

Im Gehim wird 5-Hydroxytryptamin im Perikaryon der Nervenzelle synthetisiert und über das Axoplasma den Nervenendigungen zugeführt. Eine Speicherung erfolgt in Vesikeln, aus denen das biogene Amin bei Stimulierung in den synaptischen Spalt exocytotisch freigesetzt wird.

Für die normale Reizweiterleitung durch einen Neurotransmitter ist es erforderlich, dass dieser sehr schnell wieder beseitigt wird, damit der Rezeptor für eine neuerliche Aktivierung regeneriert werden kann. Neben der Inaktivierung durch abbauende Enzyme, im Fall von Serotonin zum Beispiel Monoaminoxidase A, ist die Wiederaufnahme in die Nervenzelle die wichtigste Form der Neurotransmitter-Inaktivierung im Zentralnervensystem.

Das freigesetzte Serotonin wird daher zum Teil aus dem synaptischen Spalt durch ein spezifisches Transportprotein in der Zellmembran wieder in die serotoninerge Nervenendigung aufgenommen.

Serotonin übt seine Wirkung durch Bindung an verschiedene Zellmembranrezeptoren aus, die pharmakologisch in mindestens vier Gruppen (5-HT₁, 5-HT₂, 5-HT₃, 5-HT₄) mit zusätzlichen Subtypen untergliedert sind und an Neuronen, Gliazellen, glatter Muskulatur, Endothel- und Epithelzellen und Thrombocyten nachweisbar sind.

5-HT₁, 5-HT₂ und 5-HT₄-Rezeptoren übermitteln extracelluläre Signale über G-Proteine (G-Protein-gekoppelte Rezeptor-Grossfamilie) und vermitteln langsam eine modulierende Zellantwort über eine Beeinflussung der Adenylatcyclase (Hemmung oder Stimulierung) oder Stimulierung der Phospholipase C.

Dagegen ist der 5-HT₃-Rezeptor ein Liganden-regulierter lonenkanal, der eine schnelle Depolarisation im Neuron verursacht.

Durch die verschiedenen Rezeptoren werden unterschiedliche biologische Effekte vermittelt:

5-HT₁-Rezeptoren verursachen eine Relaxation der glatten Muskulatur in Gefässen und im Gastrointestinal-Trakt und eine selektive Kontraktion kranialer Blutgefässe,

5-HT₂-Rezeptoren bedingen eine Kontraktion der glatten Muskulatur und Plättchenaggregation,

5-HT₃-Rezeptoren sind an der Entstehung von Übelkeit, Erbrechen, Schmerzen und Angst beteiligt.

Als ursächlicher Vorgang einer Depression wird heute eine Verminderung der noradrenergen oder serotoninergen Neurotransmission, oder beider, im ZNS vermutet (Monoaminmangel-Hypothese der Depression). Der depressiven Erkrankung liegt ein Monoaminmangel zugrunde, der zu einer gesteigerten Empfindlichkeit prä- und postsynaptischer Rezeptoren für die Monoamine Noradrenalin und Serotonin führen soll.

Neben den Monoaminoxidase-Inhibitoren werden heute hauptsächlich tricyclische Antidepressiva und ihre Verwandten einschliesslich der selektiven Serotonin-Wiederaufnahme-Inhibitoren (SSRI) zur medikamentösen Behandlung von Depressionen eingesetzt.

Tricyclische Antidepressiva und ihre Verwandten hemmen die Monoamin-Aufnahme aus dem synaptischen Spalt meistens relativ unselektiv. SSRI hemmen dagegen selektiv die Rückaufnahme von Serotonin.

Durch die Hemmung der Serotonin-Transporter (Hemmung der Wiederaufnahme) wird die Konzentration des entsprechenden Neurotransmitters im synaptischen Spalt erhöht.

Durch die längerfristige Gabe von solchen Antidepressiva kann daher nach entsprechenden Adaptationsprozessen (z.B. "downregulation" von verschiedenen prä- oder postsynaptischen Rezeptoren) die noradrenerge und serotoninerge Neurotransmission im ZNS wieder normalisiert werden.

Als SSRI sind sowohl chemisch-synthetische Wirkstoffe (z.B.: Fluvoxamin, Fluoxetin und Paroxetin) als auch Pflanzenzubereitungen (z.B.: Johanniskraut-Extrakt) in Gebrauch.

Aufgrund ihres für Serotonin selektiven Wirkmechanismus und der über Serotonin-Rezeptoren vermittelten Wirkungen werden SSRI heute aber auch erfolgreich bei chronischen Schmerzen, Panikattacken, generalisierter Angststörung, Zwangssyndrom, klimakterischen Beschwerden und Eßstörungen wie Bulimie eingesetzt.

Pflanzen der Gattung Sideritis, die zur Familie der Lippenblütler (Labiaceae) gehören und von denen bisher etwa 150 verschiedene Arten bekannt sind, finden im Mittelmeerraum traditionell in der Volksmedizin bei entzündlichen Erkrankungen Anwendung. So hat bereits der griechische Arzt Dioskurides im 1. Jahrhundert nach Christus in seiner Arzneimittellehre beschrieben, dass Sideritis-Pflanzen als Umschlag angewandt "die Kraft haben, Wunden zu verkleben und Entzündungen abzuhalten".

Neuere Arbeiten aus den letzten Jahren konnten nachweisen, dass verschiedene Sideritis-Arten antibakterielle, antioxidative, analgetische und entzündungshemmende Wirkung haben.

Als Inhaltsstoffe wurden bisher Mono-, Di-, und Triterpene, Flavonoide und andere Stoffe (z.B.: Coffeinsäure) gefunden.

In der Druckschrift "Yusuf Öztürk et al.; Effects of Extracts of Certain Sideritis Species on Swimming Performance of Mice; in: Phytotherapy Research, Band 10, Seiten 70 bis 73 (1996)" wird beschrieben, daß *Sideritis*-Spezies als sedierendes und auch stimulierendes Heilmittel eingesetzt werden. Eine in der Druckschrift aus den Versuchsergebnissen abgeleitete Wirkung auf das ZNS der Versuchstiere hat jedoch nichts mit einer Wirkung auf den speziellen Neurotransmitter Serotonin (und damit zu einer serotoninergen Wirkung) zu tun und ist erst recht kein Indiz für eine Wirkung als Serotonin-Wiederaufnahmelnhibitor (SSRI).

Überraschend wurde nun gefunden, dass ein Extrakt aus getrockneten Pflanzen der Gattung Sideritis in Serotonin-Wiederaufnahme-Versuchen mit Synaptosomen aus Ratten-Cortex starke hemmende Eigenschaften zeigte.

Die Erfindung betrifft daher die Verwendung von Extrakten von Pflanzen der Gattung *Sideritis* und/oder von Teilen der Pflanzen zur Herstellung eines Arzneimittels zur Vorbeugung und/oder Beeinflussung von Störungen, die mit einer veränderten serotoninergen Neurotransmission verbunden sind gemäß Anspruch 1.

Die Erfindung betrifft somit die Verwendung von Extrakten von Pflanzen der Gattung *Sideritis* und/oder von Teilen der Pflanzen zur Herstellung eines Arzneimittels zur Vorbeugung und/oder Beeinflussung von depressiven Störungen, Panik-Attacken, generalisierten Angststörungen, Zwangssyndrom, klimakterischen Beschwerden und Essstörungen wie Bulimie.

Die Erfindung wird nachfolgend anhand bevorzugter Ausführungsformen und einzelner Beispiele, die ebenfalls bevorzugte Ausführungsformen betreffen, erläutert, ohne auf diese beschränkt zu sein.

In bevorzugten Verwendungen gemäß der Erfindung kommen Extrakte von Pflanzen von *Sideritis* zur Anwendung. Alternativ dazu ist es auch möglich, Pflanzenteile und Extrakte von *Sideritis* zusammen zu verwenden.

Wenn Extrakte von *Sideritis* verwendet werden, sind dies beispielsweise ethanolisch-wäßrige Extrakte, wobei das Volumenverhältnis der Ethanol-Wasser-Mischung für die Gewinnung der Extrakte aus *Sideritis* in weiten Grenzen schwanken kann. Es liegt besonders bevorzugt bei 99 : 1 bis 1 : 99 Vol.-%, noch weiter bevorzugt bei 65 : 35 bis 35 : 65 Vol.-%, am meisten bevorzugt bei 55 : 45 bis 45 : 55 Vol.-% (bezogen auf das Verhältnis Ethanol : Wasser in der gesamten, zur Extraktion verwendeten Mischung).

Als Extraktionsmittel können jedoch auch andere Mischungen, beispielsweise Mischungen von Wasser mit organischen Lösungsmitteln, oder auch reine Lösungsmittel verwendet werden. Infrage kommen Wasser, Dimethylsulfoxid (DMSO), Mischungen von Kohlenwasserstoffen wie beispielsweise die unter dem Begriff "Petrolether" bekannten niedrig siedenden Benzin-Fraktionen (überwiegend Pentane und Hexane als Komponenten), halogenierte Kohlenwasserstoffe, insbesondere chlorierte Kohlenwasserstoffe, beispielsweise Mischungen aus Methylenchlorid und Chloroform sowie Ester niederer (C₁- bis C₄-) Alkohole mit niederen (C₁- bis C₄-) Carbonsäuren, beispielsweise Essigester (Essigsäureethylester).

Extraktionsmittel kann auch CO₂ sein. Eine CO₂-Extraktion kann beispielsweise (jedoch nicht ausschließlich) bei überkritischen Bedingungen erfolgen, was den Vorteil hat, für die zu extrahierenden Pflanzen der Gattung *Sideritis* besonders schonend zu sein.

In weiter bevorzugten Ausführungsformen der Erfindung werden die mittels der vorstehend beispielhaft genannten Extraktionsmittel hergestellten Extrakte nicht als solche verwendet, sondem in Form von Fraktionen, die aus den genannten Extrakten gewonnen wurden. Dies kann auf beliebigen, dem Fachmann als solches bekannten Wegen geschehen und bedarf daher an dieser Stelle keiner weiteren näheren Erläuterung.

Weiter können in anderen bevorzugten Ausführungsformen gemäß der Erfindung die Extrakte von Pflanzen der Gattung *Sideritis* und/oder deren Teilen und/oder aus solchen Extrakten hergestellte Fraktionen einzeln oder in Kombination miteinander in Kombination mit anderen Pflanzen in Form von Pflanzenteilen und/oder in Form von Extrakten solcher anderer Pflanzen verwendet werden. Genauso ist auch erfindungsgemäß bevorzugt, die Extrakte von Pflanzen der Gattung *Sideritis* und/oder deren Teilen und/oder aus solchen Extrakten hergestellte Fraktionen einzeln oder in Kombination miteinander in Kombination mit chemisch-synthetischen Stoffen zu verwenden; auch im letztgenannten Fall können in einer bevorzugten Ausführungsform andere Pflanzen in Form von Pflanzenteilen und/oder Extrakten solcher anderer Pflanzen verwendet werden.

Die erfindungsgemäße Verwendung gemäß den vorstehend beschriebenen Ausführungsformen kann eine Verwendung in Form einer pharmazeutischen Zubereitung sein. Als solche kann sie allgemein übliche und dem Fachmann als solche bekannte Darreichungsformen annehmen, die eine bestimmte Konzentration an *Sideritis*-Komponenten in einer Form enthalten, die einer Verabreichung mit einem bestimmten Plan (einmal täglich eine Dosis-Einheit oder dreimal täglich eine Dosis-Einheit, um nur zwei Beispiele zu nennen) folgt. Die Darreichungsform richtet sich in dem Fachmann bekannter Weise nach dem Verabreichungsweg (oral, intravenös, intramuskulär, nasal) und kann eine feste, halbfeste, flüssige, nebelartige, gasförmige oder andere die Verabreichung auf dem gewünschten Weg erlaubende Form sein.

In einer anderen Ausführungsform kann die Verwendung eine Verwendung in Form eines Nahrungsergänzungsmittels sein. Als solche kann sie ebenfalls allgemein übliche, dem Fachmann als solche bekannte Aufnahmeformen annehmen, die üblicherweise oral aufnehmbare Formen sind. Beispiele hierfür sind meist feste, gegebenenfalls in flüssige Form bringbare (beispielsweise lösbare oder suspendierbare oder dispergierbare) feste oder flüssige Aufnahmeformen. Diese können bei der erfindungsgemäßen Verwendung allein aufgenommen werden oder zusammen mit anderen Nahrungsmitteln aufgenommen werden.

Die folgenden Beispiele einer bevorzugten Ausführungsform erläutern die Erfindung, ohne sie zu beschränken.

### Versuchsdurchführung:

Männliche Wistar-Ratten (250-300 g) wurden einzeln in einem klimatisierten Raum (21 +/- 2 °C) mit 12 h hell/dunkel-Zyklus gehalten. Die Tiere erhielten Futter und Wasser ad libitum.

Die Tiere wurden dekapitiert, und das Gehirn wurde schnell entnommen. Der Cortex wurde auf Eis herauspräpariert. Das Cortex-Gewebe wurde in 10 Volumenteilen eiskalter 0,32 M Saccharoselösung (mit 2,5 mM HEPES, pH 7,4) mit einem Potter-Elvehjem-Glas/Teflon-Homogenisator unter Eiskühlung homogenisiert, und das entstandene Homogenat wurde 10 min bei 900*g und 4 °C zentrifugiert.

Der Überstand wurde entnommen, in Eppendorf-Hütchen überführt und 10 min bei 11.000*g (4 °C) zentrifugiert. Die so präparierten Synaptosomen wurden bis zur weiteren Verwendung auf Eis gelagert.

Das verwendete Puffersystem war Farnebopuffer (121 mM NaCl, 1,8 mM KCl, 1,3 mM CaCl₂, 1,2 mM MgSO₄, 25 mM NaHCO₃, 1,2 mM KH₂PO₄, 11 mM Glucose, 0,57 mM Ascorbinsäure, gesättigt mit 95% O₂/5% CO₂, pH 7,4) mit 50 µM Pargylin (als MAO-Hemmer).

Sideritis wurde zerkleinert und in DMSO extrahiert. 10 µl dieser Lösung wurden zusammen mit 180 µl Puffer in eine Vertiefung einer 96-well Filterplatte (Millipore MultiScreenFB) gegeben. Die Endkonzentration an Sideritis war 500 µg/ml.

Für Vergleichsexperimente wurden Johanniskraut (500 µg/ml) und der selektive Serotonin-Wiederaufnahmehemmer Fluvoxamin (10 µM) eingesetzt.

Die auf Eis gelagerten Synaptosomen wurden in Farnebopuffer resuspendiert und jeweils 50 µl jedem Ansatz zugegeben. Der Ansatz wurde bei Raumtemperatur 10 min geschüttelt.

Anschließend wurden schnell 10 µl [³H]-Serotoninlösung zugegeben und der Ansatz 20 min bei 37 °C unter Schütteln inkubiert.

Die Reaktion wurde durch Absaugen und mehrfaches Spülen mit Farnebopuffer beendet. Die Radioaktivität im Filter wurde nach Herausstanzen aus der Platte durch Szintillationsmessungen bestimmt.

Die Auswertung der Versuche führte zu folgenden Wiederaufnahme-Raten (vgl. auch Figur 1):

| | |
|---|---|
| Kontrolle | 1,0000 ± 0,1014 |
| Sideritis 500 µg/ml | 0,2413 ± 0,0315 |
| Hypericum 500 µg/ml | 0,2245 ± 0,0365 |
| Fluvoxamin 10 µM | 0,1540 ± 0,0213 |

(angegeben ist jeweils der Mittelwert ± 95%-Konfidenzintervall aus 8 Versuchen).

### Literatur:

G. Löffler und P.E. Petrides; Biochemie und Pathobiochemie; Springer-Verlag Berlin Heidelberg New York; 5. Auflage 1997 und 4. Auflage 1990

W. Forth, D. Henschler, W. Rummel, K. Starke; Allgemeine und spezielle Pharmakologie und Toxikologie; Spektrum Akad. Verlag Heidelberg; Berlin; Oxford; 7. Auflage 1996

Rodriguez-Linde ME, Diaz RM, Garcia-Granados A, Quevedo-Sarmiento J, Moreno E, Onorato MR, Parra A, Ramos-Cormenzana A. Antimicrobial activity of natural and semisynthetic diterpenoids from Sideritis spp. Microbios. 1994; 77 (310): 7 bis 13

Triantaphyllou K, Blekas G, Boskou D. Antioxidative properties of water extracts obtained from herbs of the species Labiaceae, Int. J. Food Sci. Nutr. 2001 Jul; 52(4): 313 bis 317

Hernandez-Perez M, Rabanal Gallego RM. Analgesic and antiinflammatory properties of Sideritis lotsyi var. Mascaensis. Phytother Res. 2002 May; 16(3): 264 bis 266

Aboutabl EA, Nassar MI, Elsakhawy FM, Maklad YA, Osman AF, EI-Khrisy EA. Phytochemical and pharmacological studies on Sideritis taurica Stephan ex Wild. J. Ethnopharmacol. 2002 Oct; 82(2-3): 177 bis 184

Arzneimittellehre des Dioskurides: IV Buch, Cap. 36, S. 382 ff.

## Patentansprüche

1. Verwendung von Extrakten von Pflanzen der Gattung *Sideritis* und/oder von Teilen der Pflanzen zur Herstellung eines Arzneimittels zur Vorbeugung und/oder Beeinflussung von Störungen, die mit einer veränderten serotoninergen Neurotransmission verbunden sind, **dadurch gekennzeichnet, dass** es sich bei den Störungen um depressive Störungen, Panik-Attacken, generalisierte Angststörungen, Zwangssyndrom, klimakterische Beschwerden, oder Essstörungen wie Bulimie handelt.

2. Verwendung nach Anspruch 1, **dadurch gekennzeichnet, dass** es sich bei den Extrakten vorzugsweise um ethanolisch-wäßrige Extrakte und/oder Wasser-Extrakte und/oder Dimethylsulfoxid-(DMSO-) Extrakte und/oder Extrakte mit Mischungen von Kohlenwasserstoffen, vorzugsweise Petrolether-Extrakte, und/oder Extrakte mit halogenierten Kohlenwasserstoffen, vorzugsweise Extrakte mit chlorierten Kohlenwasserstoffen, besonders bevorzugt Extrakte mit Mischungen aus Methylenchlorid und Chloroform, und/oder Extrakte mit Estern niederer (C₁- bis C₄-) Alkohole mit niederen (C₁- bis C₄-) Carbonsäuren, vorzugsweise Essigester- (Essigsäureethylester-) Extrakte und/oder CO₂-Extrakte und/oder um aus Extrakten hergestellte Fraktionen, in flüssiger oder getrockneter Form, handelt.

3. Verwendung nach einem der Ansprüche 1 bis 2, **dadurch gekennzeichnet, dass** die Extrakte in Kombination mit anderen Pflanzen in Form von Pflanzenteilen und/oder Extrakten und/oder in Kombination mit chemisch-synthetischen Stoffen verwendet werden.

4. Verwendung nach einem der Ansprüche 1 bis 3, **dadurch gekennzeichnet, dass** die Verwendung eine Verwendung in Form einer pharmazeutischen Zubereitung und/oder in Form eines Nahrungsergänzungsmittels ist.

5. Verwendung nach Anspruch 1, **dadurch gekennzeichnet, dass** es sich um depressive Störungen handelt.

6. Verwendung nach Anspruch 1, **dadurch gekennzeichnet, dass** es sich um Panik-Attacken handelt.

7. Verwendung nach Anspruch 1, **dadurch gekennzeichnet, dass** es sich um generalisierte Angststörungen handelt.

8. Verwendung nach Anspruch 1, **dadurch gekennzeichnet, dass** es sich um ein Zwangssyndrom handelt.

9. Verwendung nach Anspruch 1, **dadurch gekennzeichnet, dass** es sich um klimakterische Beschwerden handelt.

10. Verwendung nach Anspruch 1, **dadurch gekennzeichnet, dass** es sich um Essstörungen handelt.

11. Verwendung nach Anspruch 1, **dadurch gekennzeichnet, dass** es sich um Bulimie handelt.

## Claims

1. Use of extracts from plants of the genus *Sideritis* and/or from parts of these plants for producing a medicament for preventing and/or influencing disorders connected with an altered serotoninergic neurotransmission, **characterized in that** the disorders are depressive disorders, panic attacks, generalized anxiety disorders, compulsive syndrome, climacteric problems or eating disorders like bulimia.

2. Use according to claim 1, **characterized in that** the extracts are preferably ethanolic aqueous extracts and/or water extracts and/or dimethyl sulfoxide (DMSO) extracts and/or extracts with mixtures of hydrocarbons, preferably petroleum ether extracts, and/or extracts with halogenated hydrocarbons, preferably extracts with chlorinated hydrocarbons, particularly preferred extracts with mixtures of methylene chloride and chloroform, and/or extracts with esters of lower (C₁ to C₄) alcohols with lower (C₁ to C₄) carboxylic acids, preferably acetic ester (acetic acid ethyl ester) extracts and/or CO₂ extracts and/or fractions prepared from extracts, in liquid or dried form.

3. Use according to one of claims 1 to 2, **characterized in that** the extracts are used in combination with other plants in form of parts of plants and/or extracts and/or in combination with chemical synthetic substances.

4. Use according to one of claims 1 to 3, **characterized in that** the use is a use in form of a pharmaceutical composition and/or in form of a dietary supplement.

5. Use according to claim 1, **characterized in that** it relates to depressive disorders.

6. Use according to claim 1, **characterized in that** it relates to panic attacks.

7. Use according to claim 1, **characterized in that** it relates to generalized anxiety disorders.

8. Use according to claim 1, **characterized in that** it relates to a compulsive syndrome.

9. Use according to claim 1, **characterized in that** it relates to climacteric problems.

10. Use according to claim 1, **characterized in that** it relates to eating disorders.

11. Use according to claim 1, **characterized in that** it relates to bulimia.

## Revendications

1. Emploi d'extraits de plantes du genre Sideritis et/ou de fragments de plantes pour fabriquer un médicament destiné à prévenir et/ou influencer des troubles liés à une neurotransmission sérotoninergique modifiée, **caractérisé en ce qu'**il s'agit de troubles dépressifs, de crises de panique, d'anxiété généralisée, de syndrome obsessionnel, de troubles climatériques ou de troubles alimentaires comme la boulimie.

2. Emploi selon la revendication 1, **caractérisé en ce que** les extraits sont de préférence des extraits éthanoliques aqueux et/ou des extraits aqueux et/ou des extraits de diméthylsulfoxyde (DMSO) et/ou des extraits avec des mélanges d'hydrocarbures, de préférence des extraits de pétrole-éther, et/ou des extraits avec des hydrocarbures halogénés, de préférence des extraits avec des hydrocarbures chlorés, en particulier de manière préférée des extraits avec des mélanges de chlorure de méthylène et de chloroforme et/ou des extraits avec des esters d'alcools inférieurs (C₁ à C₄) avec des acides carboxyliques inférieurs (C₁ à C₄), de préférence des extraits d'esters acétiques (ester éthylique de l'acide acétique) et/ou d'extraits de CO₂ et/ou de fractions fabriquées à partir d'extraits, sous forme liquide ou sèche.

3. Emploi selon la revendication 1 ou 2, **caractérisé en ce que** les extraits sont employés en combinaison avec d'autres plantes sous forme de fragments de plantes et/ou d'extraits et/ou en combinaison avec des substances chimiques synthétiques.

4. Emploi selon l'une des revendications 1 à 3, **caractérisé en ce que** l'emploi est un emploi sous forme de préparation pharmaceutique et/ou sous forme de complément alimentaire.

5. Emploi selon la revendication 1, **caractérisé en ce qu'**il s'agit de troubles dépressifs.

6. Emploi selon la revendication 1, **caractérisé en ce qu'**il s'agit de crises de panique.

7. Emploi selon la revendication 1, **caractérisé en ce qu'**il s'agit d'anxiété généralisée.

8. Emploi selon la revendication 1, **caractérisé en ce qu'**il s'agit d'un syndrome obsessionnel.

9. Emploi selon la revendication 1, **caractérisé en ce qu'**il s'agit de troubles climatériques .

10. Emploi selon la revendication 1, **caractérisé en ce qu'**il s'agit de troubles alimentaires.

11. Emploi selon la revendication 1, **caractérisé en ce qu'**il s'agit de boulimie.
